(19) Europäisches Patentamt
European Patent Office
Office européen des brevets

(11) Publication number: 0 315 491 A2

(12) # EUROPEAN PATENT APPLICATION

(21) Application number: 88310456.4

(22) Date of filing: 07.11.88

(51) Int. Cl.⁴: G 03 G 5/06
C 07 D 309/34,
C 07 D 335/02, A 61 K 31/35,
A 61 K 31/38

(30) Priority: 06.11.87 US 117585

(43) Date of publication of application:
10.05.89 Bulletin 89/19

(84) Designated Contracting States: DE FR GB

(71) Applicant: EASTMAN KODAK COMPANY
Patent Department 343 State Street
Rochester New York 14650 (US)

(72) Inventor: Boaz, Neil Warren c/o EASTMAN KODAK COMPANY
Patent Department 343 State Street
Rochester New York 14650 (US)

Chen, Chin Hsin c/o EASTMAN KODAK COMPANY
Patent Department 343 State Street
Rochester New York 14650 (US)

Chen, Lan Bo c/o EASTMAN KODAK COMPANY
Patent Department 343 State Street
Rochester New York 14650 (US)

(74) Representative: Nunney, Ronald Frederick Adolphe et al
Kodak Limited Patent Department Headstone Drive
Harrow Middlesex HA1 4TY (GB)

(54) **Amino-substituted pyrylium dyes and uses thereof.**

(57) There is disclosed a novel class of pyryliums of the 2,4,6-triaryl pyrylium dye class, having two or more of the aryl rings substituted with amino or dalkyl amino groups. These dyes have utility as sensitizers for electrophoto conductive coatings, and as drugs in treating melanoma and differentiated carcinomas in animals.

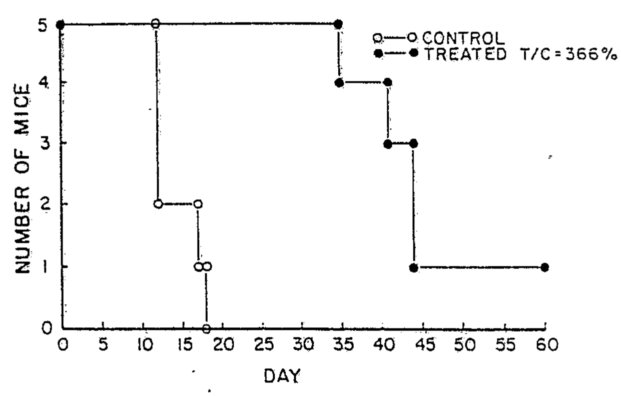


FIG. 1

EP 0 315 491 A2

## Description

# AMINO-SUBSTITUTED PYRYLIUM DYES AND USES THEREOF

This invention relates to novel pyryliums and methods of using them.

Pyryliums substituted with unsubstituted or substituted phenyls in the 2,4, & 6 position have been described heretofore. In U.S. Patent No. 4,341,894, they are described as being useful for electrophotoconductive compositions, as the sensitizers. In EPO application 88/302840.9, certain thiopyryliums are indicated as being useful in an antitumor composition, specifically in treating certain carcinomas and melanoma. (Heretofore, such compounds have also been named, thiapyryliums.)

None of those compounds identified in the above documents is a compound of this invention, namely one in which at least two of the phenyl substituents have an amino group. Some of the pyryliums heretofore described have demonstrated efficacy in treating some cancers. The problem addressed is to provide novel pyryliums having further improved efficacy in treating cancer.

In accord with one aspect of the invention, the above problem is solved by novel pyryliums having the structural formula:

I

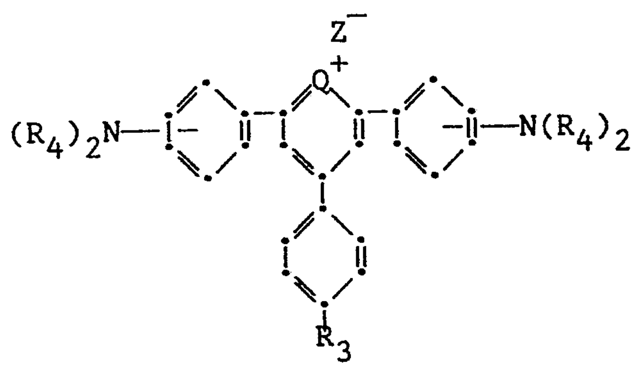

wherein

Q is O, S, or Te;

$R_3$ is H, alkyl or alkoxy of from 1 to 3 carbon atoms, or $N(R_4)_2 \bullet (HZ^1)_n$;

$R_4$ is H or an alkyl of from 1 to 3 carbon atoms;

Z and $Z^1$ are individually an anion; and

n is 0 or 1.

In accord with another aspect of the invention, the above problem is solved by a preferred class of pyryliums, having the structural forumla I′:

I'

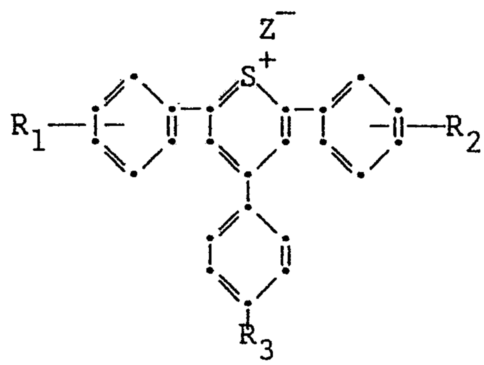

wherein
$R_1$ and $R_2$ are individually $N(R_4)_2$ . $(HZ^1)n$ or H,
$R_3$ is $N(R_4)_2$-$(HZ^1)n$, H, or alkoxy of from 1 to 3 carbon atoms, provided that at least two of the phenyl rings each have a -$N(R_4)_2$ $(HZ^1)n$ substitutent in the meta- or para- positions;
$R_4$ is $CH_3$ or H, and can be the same or different in each of $R_1$, $R_2$ and $R_3$;
Z and $Z^1$ are individually an anion; and
n is 0 or 1; with the proviso that no more than one $(HZ^1)$ group is present.

In accord with still another aspect of the invention, the above problem is solved by a method of manufacture of a medicament for the treatment of differentiated carcinomas or melanoma in a host mammalian body, comprising adding a therapeutically effective amount of a pyrylium dye sufficient to produce inhibition of growth or remission of said carcinoma or melanoma, said dye having the structure:

I'

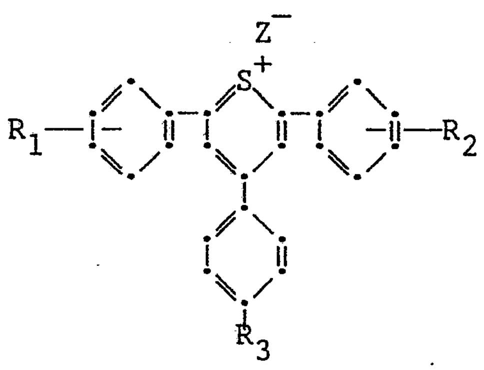

wherein
$R_1$ and $R_2$ are individually $N(R_4)_2$ • $(HZ^1)_n$ or H,
$R_3$ is $N(R_4)_2$-$HZ^1)_n$, H or alkoxy of from 1 to 3 carbon atoms, provided that at least two of the phenyl rings each have a -$N(R_4)_2$ • $(HZ^1)_n$ substituent in the meta- or para- positions;
$R_4$ is $CH_3$ or H, and can be the same or different in each of $R_1$, $R_2$ and $R_3$;
Z and $Z^1$ are individually an anion; and
n is 0 or 1; with the proviso that only one $(HZ^1)$ group is present;

to a pharmaceutically acceptable carrier.

Thus, it is an advantageous feature of the invention that the compounds of this invention have enhanced efficacy in the treatment of cancer cells in mammals, compared to thiopyryliums already existing.

The present invention will now be described by way of example with reference to the accompanying drawings in which:

Figures 1-4 are survival plots of cancer-bearing mice treated with the dyes of this invention, compared to a control group of such mice left untreated. In these plots, "% survival" means the percentage of the total group (treated "T" or control "C") still surviving on the day in question. The 50% data point is selected for the T/C calculation, as being the point that is statistically meaningful.

We have discovered that the novel pyryliums of this invention do provide enhanced properties, such as effective chemotherapeutic treatment of cancer cells. The latter treatment is particularly useful for differentiated carcinomas and melanoma. They are also useful as sensitizers for electrophotoconductive compositions.

As used herein "differentiated carcinomas" includes adenocarcinomas, which include about 90% of the prostate cancers (which occur in about 50% of the United States male population over 65); most squamous cell carcinomas; and all transitional cell carcinomas. The term does not include oat cell carcinoma or large cell carcinoma in the lung, or poorly differentiated carcinomas. Such carcinomas are not believed to be affected by this invention.

The effective treatment of differentiated carcinomas described herein produces regression and/or inhibition of growth, and remission of tumors. The specific organ cancers treatable by this invention include carcinomas of lung (except for those noted above), colon, breast, bladder, prostate, pancreas, stomach, vagina, esophagus, tongue, nasopharynx, liver, ovary, and testes.

The invention features 2,4,6-triaryl pyrylium dyes and the thio and telluro-analogs, having at least two aryls substituted with an amino group.

More specifically, the pyrylium dyes of this invention are those having the structural formula I set forth above, and most preferred are those of structural formula I′ above. For $R_1$-$R_4$, examples of alkyl includes methyl, ethyl, propyl, isopropyl, amyl and t-amyl. Examples of alkoxy include methoxy, ethoxy, propoxy, iso-propoxy, amyloxy and t-amyloxy. Depending on the selection of Q, the compounds are either pyryliums, thiopyryliums, or telluropyryliums. The dyes that are most preferred are thiopyryliums.

As used herein, "anion" is any anion that can be synthesized with the cation of the dye. For example, tetrafluoroborate and perchlorate are useful anions. "Pharmaceutically acceptable anion" means one that is nontoxic to the host mammal. Examples of useful pharmaceutical anions include halide such as chloride, bromide, iodide, and fluoride; sulfonate including aliphatic and aromatic sulfonate such as methanesulfonate, triflouromethanesulfonate, p-toluenesulfonate (tosylate), naphthalenesulfonate, and 2-hydroxyethanesulfonate; sulfamate such as cyclohexanesulfamate; sulfate such as methyl sulfate and ethyl sulfate; bisulfate; borate; alkyl and dialkyl phosphate such as dimethyl phosphate, diethyl phosphate, and methyl hydrogen phosphate; pyrophosphate such as trimethyl-pyrophosphate and diethyl hydrogen pyrophosphate; and carboxylate, preferably carboxy and hydroxy-substituted carboxylate. Preferred examples of carboxylate include acetate, propinate, valerate, citrate, maleate, fumarate, lactate, succinate, tartrate and benzoate.

For treatment of cancer cells, the most preferred anions are the halides.

Thus, useful thiopyryliums within this class include the salts formed by any of the aforesaid anions, with the thiopyryliums listed in Table I.

The thiopyrylium dyes listed in Table I are the most preferred examples of the invention.

Table I

| | |
|---|---|
| T-1 | 2,6-Bis(3-aminophenyl)-4-[4-(dimethylamino)phenyl]thiopyrylium chloride. |
| T-2 | 2,4,6-Tris(4-aminophenyl)thiopyrylium chloride. |
| T-3 | 2,6-Bis(4-aminophenyl)-4-phenylthiopyrylium chloride. |
| T-4 | 2,6-Bis(4-aminophenyl)-4-(4-methoxyphenyl)-thiopyrylium chloride. |
| T-5 | 2,6-Bis(4-aminophenyl)-4-[4-(N,N-dimethylamino)phenyl]thiopyrylium chloride. |
| T-6 | 2,4-Bis(4-dimethylaminophenyl)-6-phenylthiopyrylium perchlorate. |
| T-7 | 2,6-Bis(4-aminophenyl)-4-[4-(N,N-dimethylamino)phenyl]thiopyrylium tetrafluoroborate. |

Double salts of the above are also useful, and these occur for the structured formula I above when n = 1. For example, the dichloride can be prepared by protonating the amine nitrogen atom with hydrochloric acid.

## Synthesis

Reaction sequence II set forth below is one method of synthesizing the thiopyryliums of this invention.

II

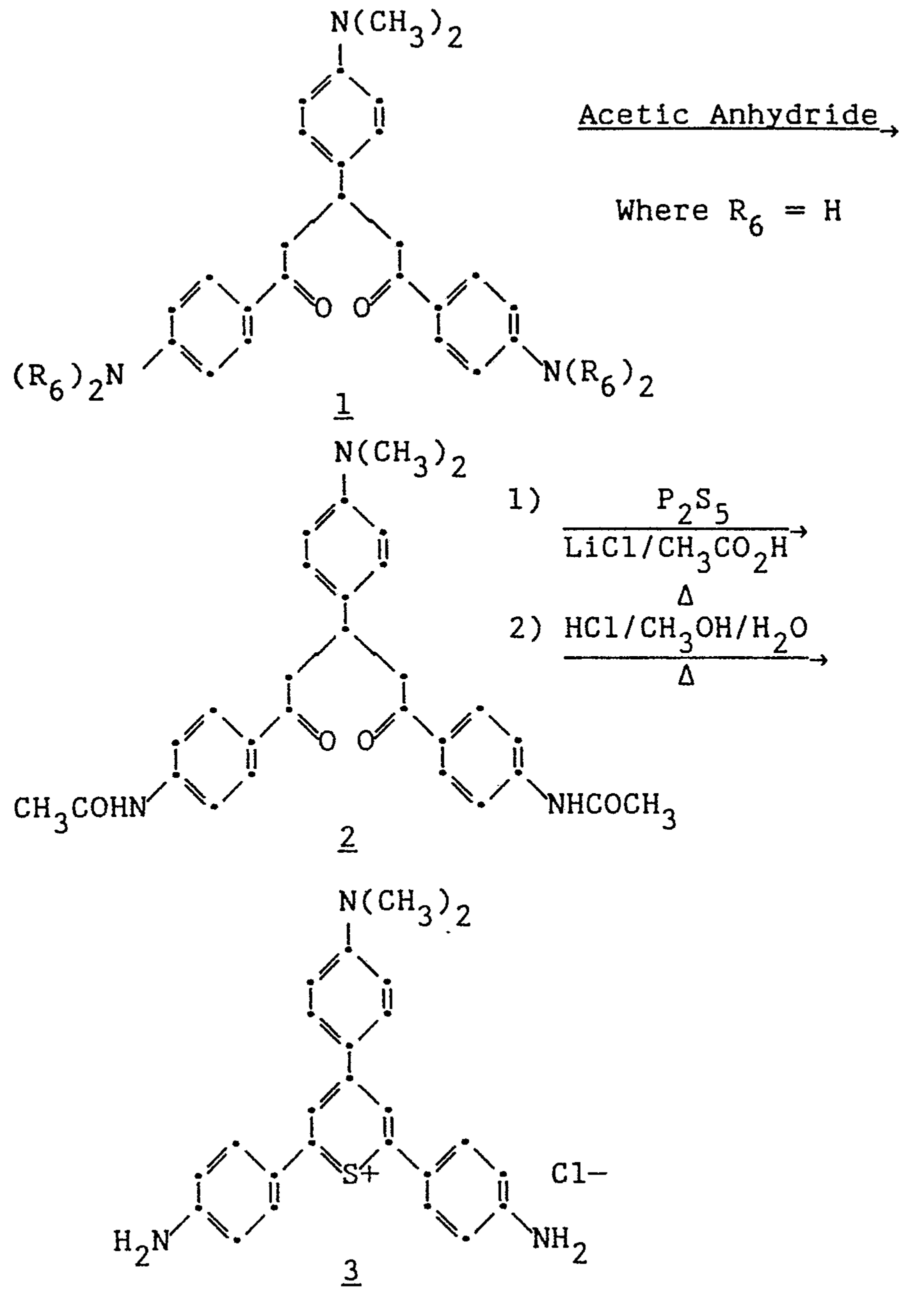


1

2

3

The following preparations are illustrative:

Preparation 1-1,5-Bis(4-aminophenyl)-3-[4-(N,N-dimethylamino)phenyl]penta-1,5-dione (Compound (1) above)

4′-Aminoacetophenone (14.97 g; 0.111 mol; 2 equiv.) and 4-N,N-dimethylamino-benzaldehyde (8.28 g; 0.056 mol) were combined and dissolved in tetrahydro furan (THF) (150 mL). Potassium tert butoxide (12.43 g; 0.111 mol; 2 equiv.) was added in one portion to give a deep red solution. The reaction mixture was stirred for 3 hours at room temperature and ammonium chloride (6 g) was added. Dichloromethane was added and the mixture was filtered to remove inorganic salts. After concentration, the residue was triturated with hot chloroform (100 mL) and filtered, and the filtrate was saved (filtrate A). The precipitate was triturated with hot chloroform (300 mL) and filtered, and the filtrate was concentrated to produce 3.43 g (15%) of (1). The solid residue (7.47 g) was shown to consist of 3.03 g (14%) of (1) and 4.444 g of inorganic salts by dichloromethane extraction from aqueous solution. Filtrate A was concentrated and then triturated with ethanol. The insoluble solid was collected and dissolved in hot chloroform (400 mL), filtered, and the filtrate was concentrated to product 6.80 g (30%) of fairly pure 1 (total yield 59%).

$^1H$ nmr (300 MHz, $CD_3CN$):
δ 7.745 (4H, d, J = 8.64 Hz);
7.090 (2H, d, J = 8.64 Hz);
6.632 (4H, d, J - 8.63 Hz);
6.628 (2H, d, J = 6.70 Hz);
4.793 (4H br s);
3.807 (1H, m(5), J = 7.29 Hz);
3.224 (2H, dd, J - 15.86, 2.11 Hz);
3.115 (2H, dd, J = 15.86, 3.15 Hz);
2.853 (6H, s);

IR (KBr):
3470 $cm^{-1}$ (m);
3360 $cm^{-1}$ (s);
3220 $cm^{-1}$ (m);
1660 $cm^{-1}$ s);
1630 $cm^{-1}$ (s);
1590 $cm^{-1}$ (s).

FDMS:
m/e 401 ($M^+$).

Preparation 2 — 1,5-Bis(4 acetamidophenyl) -3-4-(N,N-dimethylamino)phenyl]penta-1,5 dione (Compound (2) above)

1,5-Diketone 1 (2.00 g; 4.98 mmol) was placed in a 125 mL flask. Acetic anhydride (20 mL; excess) was added and the mixture was stirred for 1 hour to result in a pale tan solution which showed no (1) by TLC analysis. Water (50 mL) was added, and the mixture was stirred until homogeneity was achieved. It was then poured into 10% NaOH (200 mL) and extracted with dichloromethane (3x75 mL). The combined extracts were dried ($Na_2SO_4$ and concentrated. The residue was chromatographed through a pad of flash (32-63 mesh, 60Å) silica gel (dichloromethane application, ethyl acetate elution) to give 1.59 g (66%) of 2, leaving a red pyrylium band at the top of the column.

$^1H$ nmr (300 MHz, $CD_3CN$):
δ 8.568 (1H, br s);
7.919 (4H, d, J = 8.72 Hz);
7.658 (4H, d, J = 8.68 Hz);
7.104 (2H, d, J = 8.61 Hz);
6.639 (2H, d, J = 8.68 Hz);
3.848 (1H, m(5), J = 7.30 Hz);
3.354 (2H, dd, J = 16.39, 6.59 Hz);
3.272 (2H, dd, J = 16,37, 7.87 Hz);
2.854 (6H, s); 2.172 (6H, br s);
2.172 (6H, br s).

IR (KBr):
3320 $cm^{-1}$ (m,br);
1670 $cm^{-1}$ (s,br);
1590 $cm^{-1}$ (s).

FDMS:
m/e 485 ($M^+$);
471 ($M^+$-$CH_3$ + H).

Example 1 — 2,6-Bis(4-aminophenyl)-4-[4-(N,N-dimethyl amino)phenyl]thiopyrylium Chloride (Compound (3) above)

Protected 1,5-diketone (2) (2.50 g; 5.15 mmol), phosphorous pentasulfide (4.59 g; 20.6 mmol; 4 equiv.), and lithium chloride (1.53 g; 36.1 mmol; 7 equiv.) were combined in acetic acid (30 mL) and heated to reflux for 3 hours. The reaction mixture was filtered while hot, and the precipitate was washed with hot acetic acid and methanol. Dilution of the filtrate with ether resulted in a gummy precipitate which was collected and dried at reduced pressure. This solid was triturated with hot methanol (200 mL) and filtered, and the residue (ca. 1.2 g) was dried in vacuo. To this solid was added methanol (30 mL) and concentrated hydrochloric acid (12 mL), and the purple solution was heated to 90°C for 72 hours. After cooling, the solution was diluted with ether to provide (3) (663 mg, 30% from 2) as a purple precipitate.

$^1H$ nmr (300 MHz, $CD_3OD$):
δ 8.478 (2H, s); 8.181 (2H, d, J = 9.31 Hz);
7.932 (4H, d, J = 8.74 Hz);
7.048 (4H, d, J = 8.76 Hz);
7.000 (2H, d, J = 9.23 Hz);
3.224 (6H, s).

IR (KBr):
3390 $cm^{-1}$ (m, br);
1600 $cm^{-1}$ (m);
1550 $cm^{-1}$ (s,br);
1505 $cm^{-1}$(s).

FDMS:
398 ($M^+$);
384 (7%, $M-CH_3+H$).

UV/VIS ($CH_3OH$, $\varepsilon x10^{-3}$):
λmax 293 nm (14.1);
332 nm (13.8);
569 nm (52.0).

Yet other useful syntheses include reactions III to IV set forth below:

III

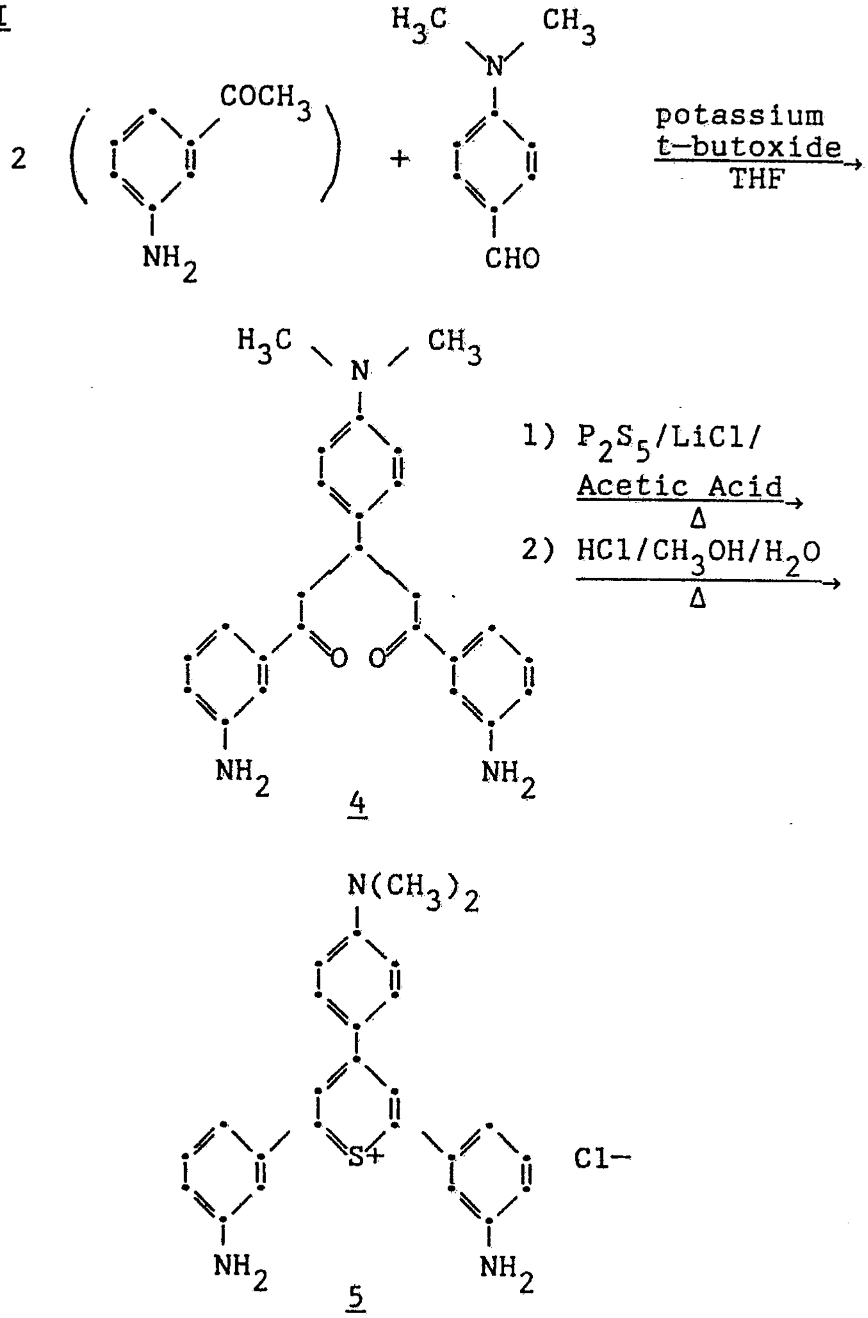
potassium
t-butoxide
THF
1) $P_2S_5$/LiCl/
Acetic Acid
Δ
2) HCl/$CH_3OH$/$H_2O$
Δ

4

5

IV

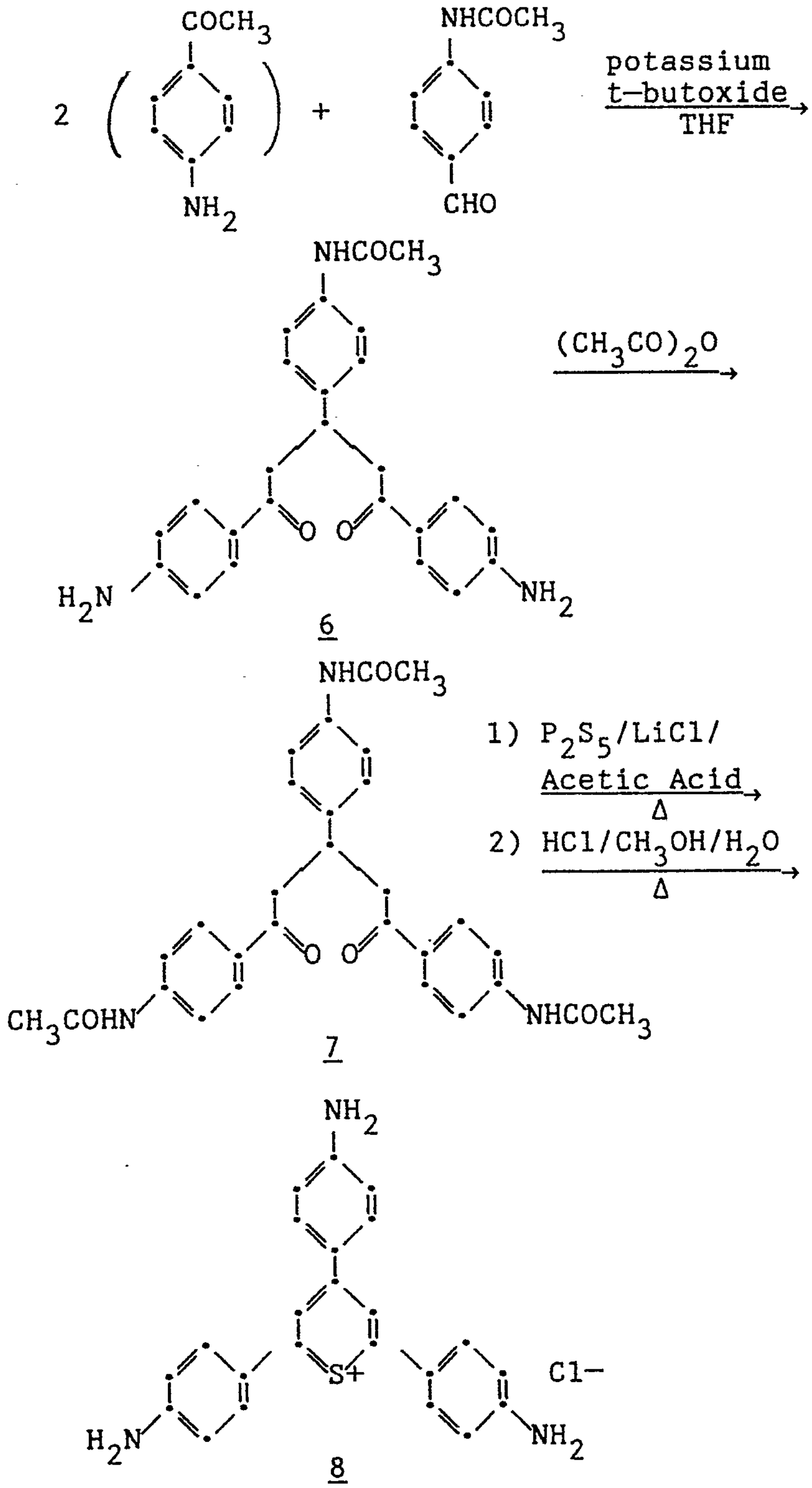
potassium
t-butoxide
THF
$(CH_3CO)_2O$
1) $P_2S_5$/LiCl/
Acetic Acid
Δ
2) $HCl/CH_3OH/H_2O$
Δ
Cl−

6

7

8

V

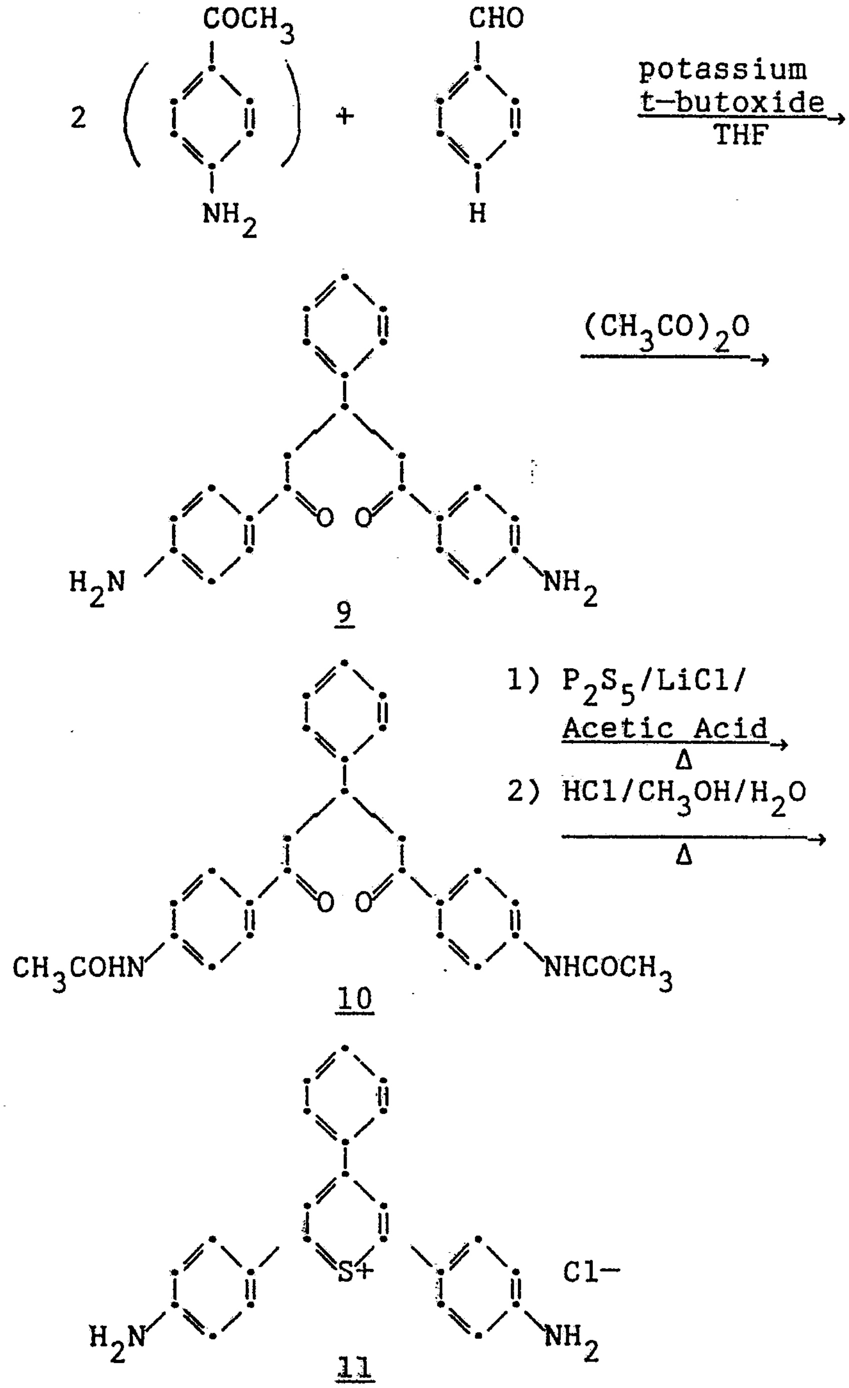
potassium
t-butoxide
THF
(CH3CO)2O
1) P2S5/LiCl/
Acetic Acid
Δ
2) HCl/CH3OH/H2O
Δ

9

10

11

VI

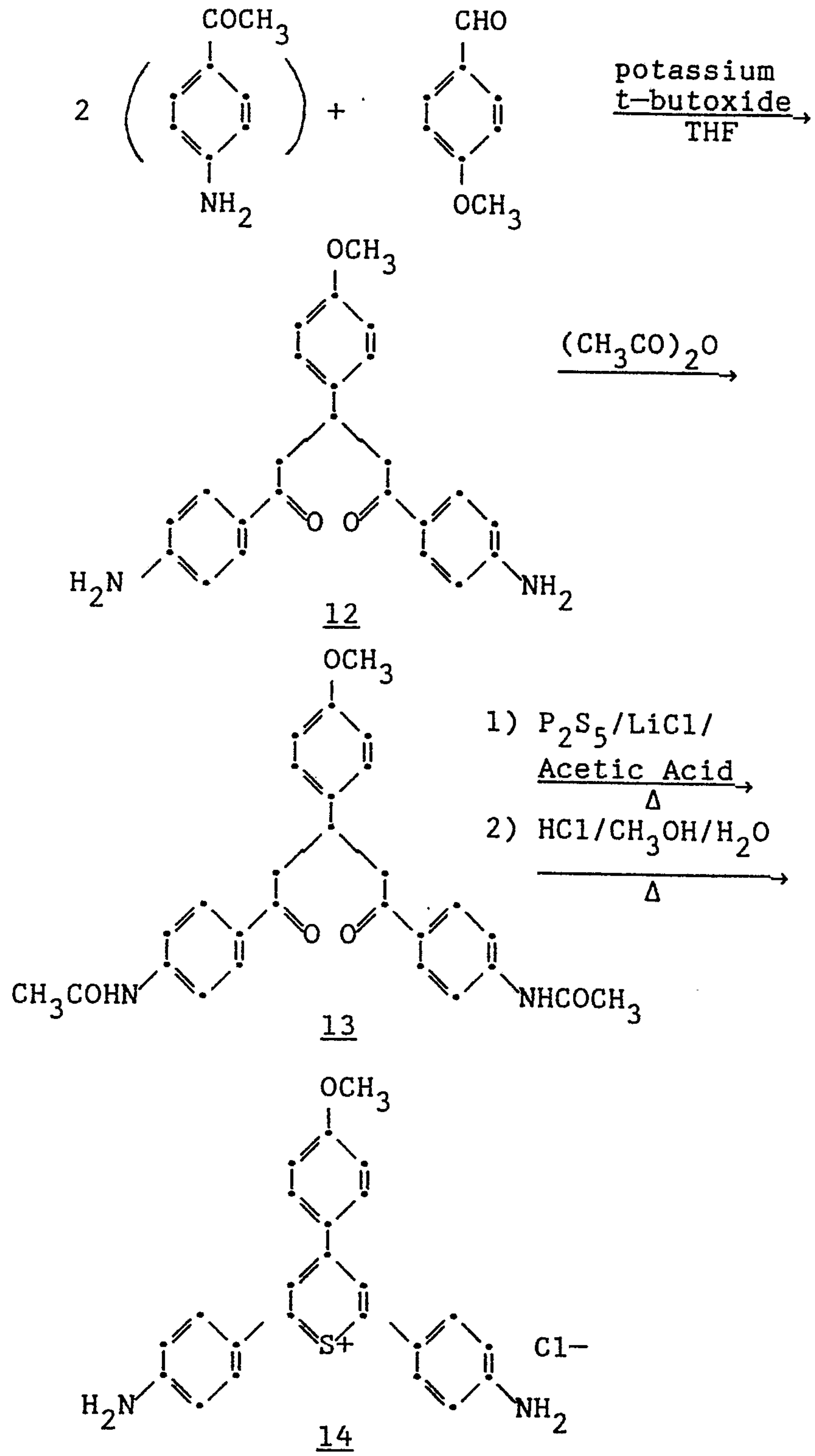
2
+
potassium
t-butoxide
THF
$(CH_3CO)_2O$
1) $P_2S_5$/LiCl/
Acetic Acid
Δ
2) $HCl/CH_3OH/H_2O$
Δ
Cl−

12

13

14

Preparation 3 — 1,5-Bis(3-aminophenyl)-3-(4-N,N-di-methylaminophenyl)penta-1,5-dione (Compound (4) in the reaction sequence above)

3′-Aminoacetophenone (10.0 g; 0.074 mol; 2.0 equiv.) and 4-N,N- dimethylaminobenzaldehyde (5.52 g; 0.037 mol) were combined and dissolved in 100 mL of THF. Potassium tert-butoxide (8.30 g; 0.074 mol; 2.0 equiv.) was added all at once. An exothermic reaction was observed and the solution changed from colorless to brownish red. The reaction mixture was stirred at room temperature for three days, at which time it was judged that equilibrium had been reached. The solvent was removed at reduced pressure and the residue was triturated with dichloromethane and filtered. The filtrate was concentrated and the residue was chromatographed on a pad of flash silica gel (described above) (2:1 ethyl acetate:hexanes eluant) Providing pure (4) (1.42 g; 10%) as well as (4) contaminated with minor by-products (4.85 g; 33%).

$^1$H nmr (300 MHz, $CDCl_3$):
7.37 (2H, d, J = 7.55 Hz);
7.26 (2H, s);
7.23 (2H, t, J = 7.80 Hz);
7.15 (2H, d, J = 8.60 Hz);
6.86 (2H, dd, J = 1.93, 7.83 Hz);
6.68 (2H, d, J = 8.62 Hz);
3.96 (1H, m(5), J = 6.96 Hz);
3.78 (4H, br s);
3.41 (2H, dd, J = 7.05, 16.25 Hz);
3.25 (2H, dd, J = 7.03 Hz, 16.27 Hz);
2.91 (6H, s).

FDMS:
m/e 401 ($M^+$).

Example 2 — 2,6 Bis(3-aminophenyl)-4-(4-N,N-di-methylaminophenyl)thiopyrylium Chloride (Compound (5) above)

The 1,5-diketone of Preparation (3)(compound (4)) (1.72 g; 4.28 mmol), phosphorous pentasulfide (3.34 g; 14.99 mmol; 3.5 equiv.), and lithium chloride (1.27 g; 29.96 mmol; equiv.) were placed in a flask and glacial acetic acid (22 mL) was added. The reaction mixture was heated to reflux for 3.5 hours and filtered while hot. The resulting precipitate was washed with acetic acid to remove all purple color and the precipitate was discarded. The filtrate was diluted with ether to provide a gummy solid which was recrystallized from methanol by ether addition to give 529 mg of a purple precipitate. This was dissolved in methanol (20 mL) and treated with concentrated hydrochloric acid (8 ml) and the mixture was heated at 90°C for 24 hours to cleave any phosphamides. Cooling to room temperature provided (5) as a blue precipitate (162 mg), which did not require further purification.

$^1$H nmr (300 MHz, DMSO-$d_6$):
8.801 (2H, s);
8.455 (2H, d, J = 9.31 Hz);
7.33 (4H, m);
7.299 (2H, s);
7.007 (2H, d, J = 8.42 Hz);
6.978 (2H, d, J = 9.32 Hz);
3.241 (6H, s).

FDMS:
m/e 398 ($M^+$);
384 (7%, $M^+$-$CH_3$+H).

UV/VIS (MeOH, $\varepsilon x10^{-3}$):
243 nm (27.4);
375 nm (9.4);
556 nm (37.6).

Preparation 4 — 1,5-Bis(4-aminophenyl)-3-(4-acetamido-phenyl)penta-1,5-dione (Compound (6) above

4′ Aminoacetophenone (5.0 g; 37 mmol; 2 equiv.) and 4 acetamidobenzaldehyde (3.02 g; 18.5 mmol) were combined and dissolved in 50 mL of THF. Potassium tert-butoxide (4.15 g; 37 mmol; 2 equiv.) was added in one portion and the reaction mixture immediately turned red. After stirring overnight at room temperature the starting materials were absent by TLC analysis and much precipitate was observed. The mixture was poured into dilute ammonium chloride solution and extracted with methylene chloride (2 X 75 mL) and 1:1 methylene chloride:acetonitrile (2 X 75 mL). The combined extracts were dried ($MgSO_4$) and concentrated to give 7.27 g

(95%) of (6) containing no observable impurities.

$^1H$ nmr (300 MHz, $CD_3CN$):
8.211 (1H, br s);
7.729 (4H, d, J = 8.45 Hz);
7.360 (2H, d, J = 8.19 Hz);
7.181 (2H, d, J = 8.28 Hz);
6.616 (4H, d, J = 8.49 Hz);
4.805 (4H, br s);
3.861 (1H, m(5), J = 7.09 Hz);
3.243 (2H, dd, J = 16.04, 5.83 Hz);
3.182 (2H, dd, J = 15.98, 8.20 Hz);
2.193 (3H, s).

FDMS:
m/e 415 ($M^+$)

Preparation 5 — 1,3,5-Tris(4-acetamidophenyl)penta-1,5-dione (Compound (7) above)

The 1,5-Diketone of Preparation 4 (Compound 6) (3.00 g; 7.22 mmol) was treated with excess acetic anhydride (30 mL) at room temperature with vigorous stirring. After 2 hours all of (6) had been consumed according to TLC analysis, and 100 mL of water was added. This mixture was stirred for 1.5 hours and then poured into 300 mL of 10% sodium hydroxide solution. This afforded a pale yellow precipitate which was collected and air-dried to give 3.22 g (89%) of (7) which was sufficiently pure for further use.

$^1H$ nmr (300 MHz, $CD_3CN$):
8.56 (2H, br s);
8.19 (1H, br s);
7.90 (4H, d, J = 8.66 Hz);
7.64 (4H, d, J = 8.45 Hz);
7.38 (2H, d, J = 8.43 Hz);
7.20 (2H, d, J = 8.45 Hz);
3.90 (1H, m(5), J = 7.20 Hz);
3.38 (2H, dd, J = 16.60, 6.59 Hz);
3.30 (2H, dd, J = 16.59, 7.75 Hz);
2.16 (6H, s);
2.09 (3H, s).

FDMS:
m/e 499 ($M^+$);
322 ($M^+$-AcNHPhCOCH$_3$).

Example 3 — 2,4,6-Tris(4-aminophenyl)thiopyrylium Chloride (Compound (8) above)

Trisacetamide (compound (7) above) (2.0056 g; 4.02 mmol), phosphorous pentasulfide (4.06 g; 18.2 mmol; 4.5 equiv.), and lithium chloride (1.2 g; 28 mmol; 7 equiv.) were combined and 25 mL of glacial acetic acid was added. The mixture was heated to reflux for 3 hours to give a red solution with precipitate. This was filtered while hot and the precipitate was washed with acetic acid. Most of the red-colored material remained in the precipitate, so it was triturated with water (50 mL) and the remaining solid was collected and air-dried. In addition, the acetic acid filtrate was diluted with ether and the resulting precipitate was collected. The solids were combined, triturated with hot methanol (75 mL), and filtered. The residual solid (623 mg) was treated with methanol (30 mL) and concentrated hydrochloric acid (12 mL; excess) and the mixture was heated to reflux for three days. After cooling to room temperature the mixture was diluted with acetonitrile and ether and the solid was collected. This was recrystallized from methanol by addition of acetonitrile and ether to provide 287 mg (18% from (7)) of (8).

$^1H$ nmr (300 MHz, DMSO-$d_6$):
8.389 (2H,s);
8.198 (2H, d, J = 8.83 Hz);
7.946 (4H, d, J = 8.68 Hz);
6.825 (6H, apparent d, J = 8.54 Hz);
5.7 (6H, br s).

FDMS:
m/e 370 ($M^+$);
355 ($M^+$-$CH_3$).

UV/VIS (MeOH, $\varepsilon x10^{-3}$):
250 nm (11.6);
279 nm (12.5);
334 nm (13.1);
535 nm (43.4).

Preparation 6 — 1,5-Bis(4-aminophenyl)-3-phenylpenta-1,5-dione (Compound (9) above) (Reaction Scheme V)

4′-Aminoacetophenone (5.0 g; 37 mmol; 2 equiv.) and benzaldehyde (1.96 g; 18.5 mmol) were dissolved in 25 mL of THF. Potassium tert-butoxide (4.15 g; 37 mmol; 2 equiv.) was added in one portion and washed in with 25 mL of THF. The reaction mixture immediately turned red and after stirring overnight at room temperature very little starting material was observed by TLC analysis. The solvent was removed at reduced pressure and the residue was partitioned between water (40 mL) and ethyl acetate (40 mL). The layers were separated and the aqueous layer was extracted with additional ethyl acetate (2 X 40 mL). The organic solutions were combined and dried ($MgSO_4$), and the solvent was removed to give 6.64 g (100%) of compound (9) which was sufficiently pure for further use.

$^1H$ nmr (300 MHz, $CD_3CN$):
7.744 (4H, d, J = 8.60 Hz);
7.24 (5H, m);
6.631 (4H, d, J = 8.63 Hz);
4.803 (4H, br, s);
3.93 (1H, m(5) J = 7.2 Hz);
3.266 (4H, m).

Preparation 7 — 1,5-Bis(4-acetamidophenyl)-3-phenylpenta-1,5-dione (Compound (10) above)

The 1,5-diketone of Preparaton 6 (Compound 9) (6.51 g; 18.5 mmol maximum) was treated with excess acetic anhydride (38 mL) to give a pale red solution. The mixture was stirred for 3 hours at room temperature during which time the solution turned to a yellow slurry. Water (100 mL) was added and the mixture was stirred for 2 hours and poured into 10% sodium hydroxide solution (400 mL). The resulting yellow precipitate was filtered and air-dried to give 6.96 g (85%) of (10), which was pure by nmr analysis, and was used as is.

$^1H$ nmr (300 MHz, DMSO-$d_6$):
10.363 (2H, s);
7.890 (4H, d, J = 8.68 Hz);
7.683 (4H, d, J = 8.70 Hz);
7.294 (2H, d, J = 7.43 Hz);
7.208 (2H, t, J = 7.32 Hz);
7.101 (1H, t, J = 7.2 Hz);
3.86 (1H, m(5), J = 6.5 Hz);
3.38 (4H, m);
2.075 (6H, s).

FDMS:
m/e 358 ($M^+$).

Example 4 — 2,6-Bis(4-aminophenyl)-4-phenylthiopyrylium chloride (Compound (11) above)

Protected 1,5-diketone (10) (5.0 g; 11.3 mmol), phosphorous pentasulfide (10.1 g; 45.2 mmol; 4 equiv.), and lithium chloride (3.35 g; 79.1 mmol; 7 equiv.) were combined in a flask and suspended in 60 mL of glacial acetic acid. The reaction mixture was heated to reflux for 3 hours to result in a red solution, which was filtered while hot. The precipitate was washed with acetic acid and the combined filtrate was diluted with ether to provide a gummy precipitate. This was collected and recrystallized from methanol by the addition of acetonitrile (1 part) and ether (5 parts). This afforded 2.087 g of the protected thiopyrylium salt, 1.80 g (3.79 mmol) of which was dissolved in methanol (75 mL) and concentrated hydrochloric acid (30 mL; excess). The mixture was heated to reflux for 72 hours to cleave the acetamides and then cooled to room temperature. The Purple solution was diluted with acetonitrile and ether and the resulting precipitate was collected [1.156 g; 30% from (10)] and dried.

$^1H$ nmr (300 MHz, DMSO-$d_6$):
8.43 (2H, s);
8.16 (2H, d, J = 7.09, 0.99 Hz);
8.03 (4H, d, J = 8.81 Hz);
7.66 (3H, m);
6.79 (4H, d, J = 8.77 Hz);
4.1 (4H, br, s).

FDMS:
m/e 355 ($M^+$);
367 (7%, $M^+$-H+$CH_3$).

UV/VIS (MeOH, $\varepsilon x10^{-3}$):
347 nm (29.3);
469 nm (7.8);
613 nm (30.6).

Preparation 8 1,5-Bis(4-aminophenyl)-3-(4-methoxyphenyl)penta-1,5-dione (Compound (12) above)
4′-Aminoacetophenone (5.0 g; 37 mmol; 2.0 equiv.) and 4 methoxybenzaldehyde (2,51 g; 18.5 mmol) were dissolved in THF (25 mL) at room temperature. Potassium tert-butoxide (4.15 g; 37 mmol; 2.0 equiv.) was added with vigorous stirring and washed in with 25 mL of THF. The solution immediately turned a deep red color and, after stirring overnight at room temperature very little starting material was observed by TLC analysis. The solvent was removed at reduced pressure and the residue was partitioned between water (40 mL) and ethyl acetate (40 mL). The layers were separated and the aqueous phase was extracted with ethyl acetate (2 X 40 mL). The combined organic solution was dried ($MgSO_4$) and the solvent was removed to provide 6.92 g (96%) of (12) which was sufficiently pure for further use ($^1$H nmr analysis).

$^1$H nmr (300 MHz, $CD_3CN$):
7.742 (4H, d, J = 8.62 Hz);
7.188 (2H, d, J = 8.60 Hz);
6.790 (2H, d, J= 8.60 Hz);
6.631 (4H, d, J = 8.58 Hz);
4.799 (4H, br s);
3.879 (1H, m(5), J = 7.06 Hz);
3.732 (3H, s);
3.241 (2H, dd, J = 16.40, 7.05 Hz);
3.180 (2H, dd, J = 16.14, 7.84 Hz).

FDMS:
m/e 388 ($M^+$).

Preparation 9 — 1,5-Bis(4-acetamidophenyl)-3-(4-methoxyphenyl)penta-1,5-dione (Compound (13) above)
The 1,5-diketone of Preparation 8 (Compound 12) (6.34 g; 16.3 mmol) was placed in a 500 mL flask and acetic anhydride (42 mL; excess) was added. The mixture was stirred for 3 hours at room temperature to give a red solution which contained no (12) by TLC analysis. Water (100 mL) was added and the mixture was stirred for 3 hours and poured into 10% aqueous sodium hydroxide (400 mL). This resulted in a red intractable gum, which was used directly for the next step without further purification.

Example 5 — 2,6-Bis(4-aminophenyl)-4-(4-methoxy phenyl)thiopyrylium chloride (Compound (14) above, reaction sequence VI)
The gummy protected 1,5-diketone (13) (2.40 g; 5.08 mmol) was combined with phosphorous pentasulfide (4.53 g; 20.3 mmol; 4.0 equiv.) and lithium chloride (1.51 g; 35.5 mmol; 7.0 equiv.). Glacial acetic acid (30 mL) was added and the mixture was heated to reflux for 3 hours to give a red solution. This solution was filtered while hot and the collected solid was washed with acetic acid. The combined filtrate was diluted with ether to provide a gummy precipitate which was recrystallized from methanol by the addition of acetonitrile and ether to give two crops (total 928 mg) of the protected thiopyrylium. Of this, 570 mg was dissolved in methanol (25 mL) and concentrated hydrochloric acid (10 mL; excess) was added. The mixture was heated to reflux for 72 hours to cleave the acetamides and then cooled to room temperature. Acetonitrile and ether were added to precipitate (14), which was collected and dried [414 mg; 31% from (13)].

$^1$H nmr (300 MHz, DMS-$d_6$):
8.431 (2H, s);
8.254 (2H, d, J = 8.76 Hz);
8.004 (4H, d, J = 8.84 Hz);
7.187 (2H, d, J = 8.83 Hz);
6.787 (4H, d, J = 8.79 Hz);
3.911 (3H, s).

FDMS:
m/e 385 ($M^+$);
370 ($M^+$-$CH_3$).

UV/VIS (MeOH, $\varepsilon x10^{-3}$):
235 nm (16.8);
394 nm (26.4);
nm (17.2);
595 nm (31.8).

## Utility Examples

The electrophotoconductive utility is practiced as described in the aforesaid U.S. Patent 4,341,894. As this utility is well-known in the art, there is no need for further elaboration.

In the method of treating differentiated carcinomas or melanoma in mammals, the dye is particularly useful when added to a pharmaceutically acceptable carrier. The pharmaceutically acceptable carrier can be any carrier, such as a solvent that will sufficiently dissolve the thiopyrylium dye. Among preferred examples of a suitable carrier solvent for human usage are a 5% dextrose solution in water, or a mixture of ethanol and a polyol such a polyethoxylated caster oil, available from the National Cancer Institute as "Diluent No. 12."

Still other acceptable carrier solvents include, dimethyl sulfoxide (DMSO) for intravesical treatment, and isotonic saline for IV and IP injections.

Still other carriers that are useful include the following:

Materials such as gelatin, natural sugars such as sucrose or lactose, lecithin, pectin, starch (for example cornstarch), alginic acid, tylose, talc, lycopodium, silica (for example colloidal silica), glucose cellulose, cellulose derivatives, for example cellulose ethers in which the cellulose hydroxyl groups are partially etherified with lower aliphatic alcohols and/or lower saturated oxyalcohols (for example, methyl hydroxypropyl cellulose, methyl cellulose, hydroxyethyl cellulose), stearates, e.g., methyl stearate and glyceryl stearate, magnesium and calcium salts of fatty acids with 12 to 22 carbon atoms, especially saturated acids (for example, calcium stearate, calcium laurate, magnesium oleate, calcium palmitate, calcium behenate and magnesium stearate), emulsifiers, oils and fats, especially of plant origin (for example, peanut oil, castor oil, olive oil, sesame oil, cottonseed oil, corn oil, wheat germ oil, sunflower seed oil, cod liver oil), mono-, di-, and triglycerides of saturated fatty acids ($C_{12}H_{24}O_2$ to $C_{18}H_{36}O_2$ and their mixtures), e.g., glyceryl monostearate, glyceryl distearate, glyceryl tristearate, glyceryl trilaurate), pharmaceutically compatible mono-, or polyvalent alcohols and polyglycols such as glycerine, mannitol, sorbitol, pentaerythritol, ethyl alcohol, diethylene glycol, triethylene glycol, ethylene glycol, propylene glycol, dipropylene glycol, poly(ethylene glycol), and other polyalkylene glycols, as well as derivatives of such alcohols and polyglycols, esters of saturated and unsaturated fatty acids (2 to 22 carbon atoms, especially 10 to 18 carbon atoms), with monohydric aliphatic alcohols (1 to 20 carbon atom alkanols), or polyhydric alcohols such as glycols, glycerine, diethylene glycol, pentaerythritol, sorbitol, mannitol, ethyl alcohol, butyl alcohol, octadecyl alcohol, etc., e.g., glyceryl stearate, glyceryl palmitate, ethylene distearate, ethylene dilaurate, ethylene diacetate, monoacetin, triacetin, glyceryl oleate, esters of polyvalent alcohols that are etherified, benzyl benzoate, dioxolane, glycerin formal, tetrahydro furfuryl alcohol, polyglycol ethers of l to 12 carbon atom alcohols, lactamide, lactates, e.g., ethyl lactate, ethyl carbonate, silicones (especially medium viscosity dimethyl polysiloxane), magnesium carbonate and the like.

Still other additives, and methods of preparation of the composition, can be found in the extant literature, for example, U. S. Patent 4,598,091 issued on July 1, 1986. Useful methods of delivery of the dye and carrier include implanted drug pump, intravenous (IV) intraperitoneal (IP) and intravesical injection.

The dosage levels depend upon which thiopyrylium dye is being used on which differentiated carcinoma (or melanoma). Such dosage may be determined by one skilled in the art, using the techniques described in Goodman and Gilman's "The Pharmacological Basis of Therapeutics" (6th edition), pages 1675-1737, subtitled "Design and Optimization of Dosage Regimens" (Macmillan Publishing Co., New York, 1980). Based on dosages commonly experienced for chemotherapeutic agents, and the correlation that has been shown between clinical tests and the $LD_{50}$ dosages found in the nude mice protocol described hereinafter, it is estimated the dosages for human consumption would be: IV injection of 150 to 300 mg/m$^2$ every 3 weeks, 5 to 10 mg/kg daily for 4 to 8 days, or 70 to 90 mg/m$^2$ daily for 3 days or once weekly for 6 weeks. ("Mg/m$^2$" refers to milligrams per square meter of the patient's body, as is customary.)

## Chemotherapy Examples

Effectiveness against human cancer can be inferred by tests in the nude mouse-human xenograft model, hereinafter "nude mice protocol". This has been suggested in, e.g., the Journal of the National Cancer Institute, Vol. 74, No. 4, p. 899-903, especially p. 889 (April 1985).

Nude mice, that is, those deficient in thymic functions, bred and maintained in pathogen free conditions [see J. Natl. Cancer Inst. 51:615-619 (1973)] were used in in vivo experiments. For each experiment, nude mice from the same litter and of the same sex which were over 3 months old and weighed at least 25g were injected or implanted with cancer cells.

Examples 6-7 — Melanoma Tests Using Nude Mice Protocol

A human melanoma cell line, LOX, was used that grows rapidly when inoculated into the peritoneal cavity of the athymic mice. Tumors which had been passaged subcutaneously in athymic mice were excised under sterile conditions and converted to a single cell suspension using a metal grid with a 4 mm mesh. Red blood cells were then lysed by incubation with 0.17 molar ammonium chloride at 4° C for 30 minutes. Cells were then scored for viability with trypan blue and 5 million viable cells made up in 0.2 ml of Dulbecco modified Eagles' medium (DME) were injected intraperitoneally (IP) into a group of male athymic Swiss nu/nu mice (day 0). The mice were then randomly allocated into treatment and control groups (5% dextrose) and treatment, by intraperitoneal injection commenced the following day.

In Example 6, five mice received 5 mg/kg of the drug T-5, Table I, administered Monday, Wednesday & Friday of each week for a total of 11 injections. Five mice received the same volume of the control 5% dextrose solution on the same days.

Fig. 1 illustrates the results for Example 6: T/C = 366%, indicating a very high improvement in survival, and remission of the tumors (the accepted point of improvement in the industry is any value of T/C > 145%).

In Example 7, the preparation of the mice and the treatment were the same as in Example 6, except that T-5 was administered at a 8 mg/kg level, starting on Wednesday as Day 1, and then on Saturday (and Wednesday and Saturday) for 3 weeks. The control mice received the dextrose solution only. The results are shown in Fig. 2. Survival was 338%, again a marked increase over the control group.

Examples 7-8 — Ovarian Carcinoma (OVCAR-3) Using Nude Mice Protocol

Examples 6 and 7 were repeated, except they were administered to female nude mice (aged 8-12 weeks) xenografted with human ovarian carcinoma line OVCAR-3. Eight mice in Example 6, and 8 mice in Example 7 were selected for treatment with the thiopyrylium, in an amount of 2.5 and 5 mg/kg respectively, administered IP 1 day after the cancer cells were injected. This was repeated as follows: Three injections per week for 6 weeks. Eight mice were used as the control group and were injected with 0.25 ml of 5% dextrose in water.

The results appear in Figure 3. The dye gave, in both cases, a T/C > 450%, which is substantially improved in efficacy, compared to other thiopyryliums.

Examples 8-9 — Treatment of Mouse Bladder Carcinoma Using Thiopyrylium Dyes Administered IP

MB49 tumor cells, that is, mouse carcinoma cells induced by the carcinogen 7,12-dimethyl benz[a]anthracene, were obtained by conventional processes and injected intraperitoneally into normal male BDFI mice (5 million/mouse). Each drug treated group and control group had 5 mice. In example 8, 5 mg/kg of T-5 was administered 3 times per week for three weeks, starting Day 1. In Example 9, it was 3 mg/kg three times per week for 3 weeks. The results were, Example 8, a T/C of 244%, and Example 9, T/C of 344%, as shown in Figure 4.

A similar experiment done with thiopyrylium T-6 in Table I demonstrated a 21.6% average inhibition in tumor growth compared to tumor growth in control animals 21 days after tumor initiation.

These values were calculated by dividing the difference between the mean weight of bladders from controls (water-treated animals) and mean weight of bladders of drug-treated animals by the mean weight of bladders from control animals and multiplying this fraction by 100. (In this experiment, the drug was administered by mixing the MB49 tumor cells ($4 \times 10^6$) with 0.05 mg/ml of the drug prior to instillation of the cell suspension into the mouse bladder.)

Comparative Examples 1-2

Following procedures that are very similar to those set forth above, 4-(4-N,N-dimethylaminophenyl-2-phenyl-6-(2-pyridyl)thiopyrylium chloride was tested against the xenografted human melanoma, and ovarian cancers. This compound is similar to T-5 of this application except that the 6 substituent was a pyridyl rather than a phenyl ring, and both the 2-and 6- ring substituents lacked an amino group. The results (also listed in the aforesaid "Related Application") were a T/C of 227% against melanoma, and T/C of 340% against the ovarian carcinoma.

Although these results are themselves evidence of significant remission, they are not as good as the 366% and the > 450% results, respectively, obtained for T-5 of this invention. It is clear from this comparison that the thiopyryliums of this application have greater efficacy against melanoma and differentiated carcinomas. This in turn is believed to result from the increased solubility provided by the amino substituents.

## Chemotherapeutic Examples

Examples 10-13 — Other Thiopyryliums

Although these examples were not tested in animal models, their similar efficacy in human melanoma and differentiated carcinomas is predicated from the fact they had similar in vitro results compared to T-5. The in vitro tests were run as follows, to ascertain the $IC_{50}$ concentrations. ($IC_{50}$ is the in vitro concentration that kills one-half the cell line against which the compound is tested. The smaller the concentration, the higher the toxicity.)

For the $IC_{50}$ values for the human lung carcinoma line, an adhering monolayer culture was used. Two days prior to plating, a number of cells obtained from the American Type Tissue Culture Collection was selected to grow as a stock, the number being that which would ensure the cells had not reached their plateau at the time of the in vitro test. On day 1 of the assay, asynchronous exponentially growing cells from this stock were suspended in a conventional complete medium comprising RPMI 1640 medium supplemented with 5% (v/v) fetal calf serum and 5% (v/v) NV serum obtained from Collaborative Research, and 20mM HEPES buffer, i.e., 4-(2-hydroxyethyl)-1-piperazineethanesulfonic acid. This was plated into a conventional 96-well tissue culture plate using a Cetus/Perkin Elmer automated pipetting device with a dual supply manifold. The cells were then incubated for 48 hours at 37°C in a humidified atmosphere containing 5% $CO_2$. Selected cells were then incubated under red light for 3 hours in the presence of varying amounts of the compound of interest at 37°C in a humidified incubator containing 5% $CO_2$. Compounds were tested over a 6-log range of concentrations. The drug treated cells were then rinsed one time with complete medium as set forth above, and incubated in complete medium the length of time appropriate to achieve four cell-doubling times (roughly 7 days for these cell lines). The medium was removed and the cells stained for 1-2 hours at 37°C with 0.1% (w/v) neutral red dye obtained from Eastman Kodak Co., dissolved in phosphate buffered saline (PBS) (without calcium or magnesium cations) at a pH of 6.85. The cells were rinsed twice with the same PBS and dried. Excess neutral red dye was eluted from each well with 50% (v/v) ethanol in 1% (v/v) aqueous glacial acetic acid. The absorbency of each well was quantitated using a Perkin-Elmer absorbence densitameter set at 540 nm. The percent of inhibition of cell growth for a given concentration of compound was calculated using the equation:

$$F(x) = \%\ \text{inhibition} = \left[1 - \frac{At - Ab}{Au - Ab}\right] \times 100$$

where
At = absorbence of the cells treated with the candidate drug,
Au = absorbence of the control treated with solvent only, and
Ab = absorbence of the media and well only (background).
These results were plotted as F(x) versus the log concentration of the test compound. The plotted data was then fitted to the curve for the function:
$F(x) = 100 - [100(X/A)^B/(1+(X/A)^B)]$
where
x = the concentration of the test compound,
A = the $IC_{50}$ value (50% inhibition on the plot) and
B = the exponent of the fitted function.

To determine the $IC_{50}$ for the L1210 leukemia cells, a suspension culture was used. The procedure for the monolayer culture was followed, except that the cells were seeded at a density of $3 \times 10^4$ cells/ml in the RPMI 1640 medium supplemented this time with 10% (v/v) NV SERUM, no calf serum, and 20mM HEPES buffer, with the compound of interest already added. These were added immediately to a conventional 24-well culture plate and incubated for a four cell doubling time (48 hours for this cell line). Cell numbers were quantitated using the Model ZF Coulter Counter. F(x) was obtained from the equation:

$$F(x) = \%\ \text{inhibition} = \left[1 - \frac{Nt - No}{Nu - No}\right] \times 100$$

where
Nt = number of treated cells detected, never less than No,
Nu = number of cells detected in the control that were untreated except for the solvent, and
No = number of cells initially seeded.

Table II sets forth the results:

Table II of $IC_{50}$ Values

| Example | Compound | L1210 Mouse Lymphoma (μ Molar) | Lung Adeno-carcinoma (μ Molar) |
|---|---|---|---|
| 6 (for comparison, tested twice | T-5: 2,6-Bis(4-aminophenyl)-4-[4-(N,N-dimethylamino)phenyl]thiopyrylium chloride | 0.22, 0.14 | 0.04, 0.06-0.08 |
| 10 | T-1: 2,6-Bis(3-aminophenyl)-4-[4-(dimethylamino)phenyl]thiopyrylium chloride | 0.038 | 0.37 |
| 11 | T-2: 2,4,6-Tris(4-aminophenyl)thiopyrylium chloride | 0.27 | 0.19 |
| 12 | T-3: 2,6-Bis(4-aminophenyl)-4-phenylthiopyrylium chloride | 0.033 | 0.017 |
| 13 | T-4: 2,6-Bis(4-aminophenyl)-4-(4-methoxyphenyl)-thiopyrylium chloride | 0.010 | 0.011 |

(the smaller the number, the more effective or toxic the dye is.)

Electrophotographic Examples 14-15

Electrophotographic elements were prepared having a photoconductive layer comprising 79.5% of Vitel PE 101 polyester sold by Goodyear Tire and Rubber Company as a binder, 20 percent of an organic photoconductor (electron donor), and 0.5% of the thiopyrylium salt sensitizer (electron acceptor) to be tested, applied at a dry thickness of 8μm to a conductive support of poly(ethylene terephthalate) coated with the sodium salt of a carboxy ester lactone of a maleic anhydride and vinyl acetate copolymer.

The photoconductors employed were (A) triphenylamine and (B) 4,4′-bis(diethylamino)-2,2′-dimethyltriphenylmethane. The sensitizer tested was 2,6-bis(4-aminophenyl)-4-[4-(N,N-dimethylamino)phenyl] thiopyrylium tetrafluoroborate (T-7 of Table I).

The films were positively charged to an initial voltage, Vo, and exposed to monochromatic light of 580 nm having an intensity of 25 ergs/$cm^2$/sec until the initial voltage was reduced by 200 volts. The sensitivity of the elements was reported as the "Monochromatic Exposure Requirement," i.e.,the exposure required to produce (Vo-200) volts in ergs/$cm^2$. The results appear in Table III below.

Table III

| Ex. | Photo-conductor | λ (nm) | Vo | Final Voltage | Monochromatic Exposure Requirements (Ergs/$Cm^2$) |
|---|---|---|---|---|---|
| 14 | A | 580 | 600 | 400 | 238 |
| 15 | B | 580 | 500 | 300 | 113 |

Control coatings having no sensitizer would take too long to discharge at 580 nm for practical measurement. Thus the data show sensitization of the photoconductors A and B by the thiopyrylium sensitizer at this wavelength.

Others of the thiopyryliums of this invention can be assumed to produce comparable sensitization of electrophotoconductive coatings, due to this property being common of the class as a whole, as shown in the aforesaid U.S. Patent No. 4,341,894.

**Claims**

1. A pyrylium having the following structured formula:

I

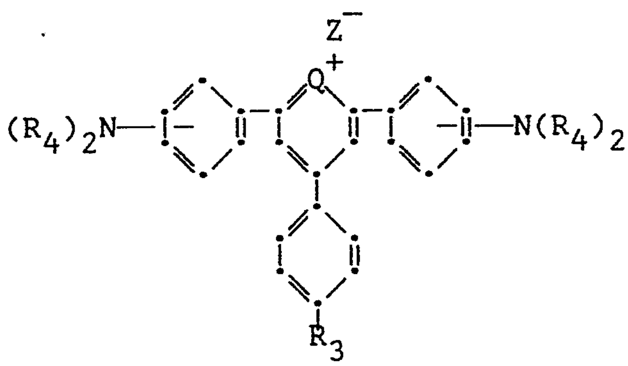

wherein
Q is 0, S, or Te;
$R_3$ is H, alkyl or alkoxy of from 1 to 3 carbon atoms, or $N(R_4)_2 \bullet (HZ^1)_n$;
$R_4$ is H or an alkyl of from 1 to 3 carbon atoms;
Z and $Z^1$ are individually an anion; and
n is 0 or 1.

2. A pyrylium salt or dye having the following structural formula:

I'

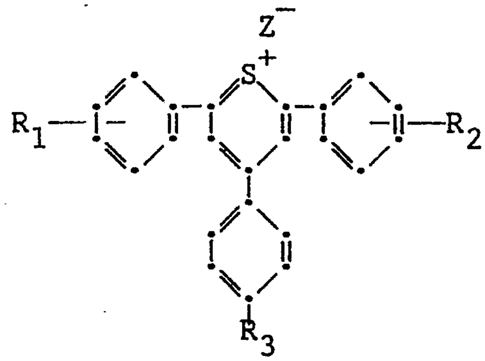

wherein
$R_1$ and $R_2$ are individually $N(R_4)_2 \bullet (HZ^1)_n$ or H,
$R_3$ is $N(R_4)_2$-$(HZ^1)_n$, H or alkoxy of from 1 to 3 carbon atoms, provided that at least two of the phenyl rings each have a-$N(R_4)_2 \bullet (HZ^1)_n$ substituent in the meta- or para- positions;
$R_4$ is $CH_3$ or H, and are the same or different in each of $R_1$, $R_2$ and $R_3$;
Z and $Z^1$ are individually an anion; and
n is 0 or 1; with the proviso that no more than one $(HZ^1)$ group is present.

3. 2,6-Bis(3-aminophenyl)-4-[4-(N,N-dimethylamino)phenyl]thiopyrylium chloride.
4. 2,4,6-Tris(4-aminophenyl)thiopyrylium chloride.
5. 2,6-Bis(4-aminophenyl)-4-phenylthiopyrylium chloride.
6. 2,6-Bis(4-aminophenyl)-4-(4-methoxy phenyl)thiopyrylium chloride.
7. 2,6-Bis(4-aminophenyl)-4-[4-(N,N-dimethylamino)phenyl]thiopyrylium chloride.
8. 2,4-Bis(4-N,N-dimethylaminophenyl)-6-phenylthiopyrylium perchlorate.
9. 2,6-bis(4-aminophenyl)-4-[4-(N,N-dimethylamino)phenyl]thiopyrylium tetrafluoroborate.
10. A pyrylium as defined in any of the preceding claims, for use as an active therapeutic substance.
11. A method of manufacture of a medicament for the treatment of differentiated carcinomas or melanoma in a host mammalian body, comprising adding a therapeutically effective amount of a pyrylium dye sufficient to produce inhibition of growth or remission of said carcinoma or melanoma to a pharmaceutically acceptable carrier, characterised in that the pyrylium dye is as defined in any one of claims 2 to 9.

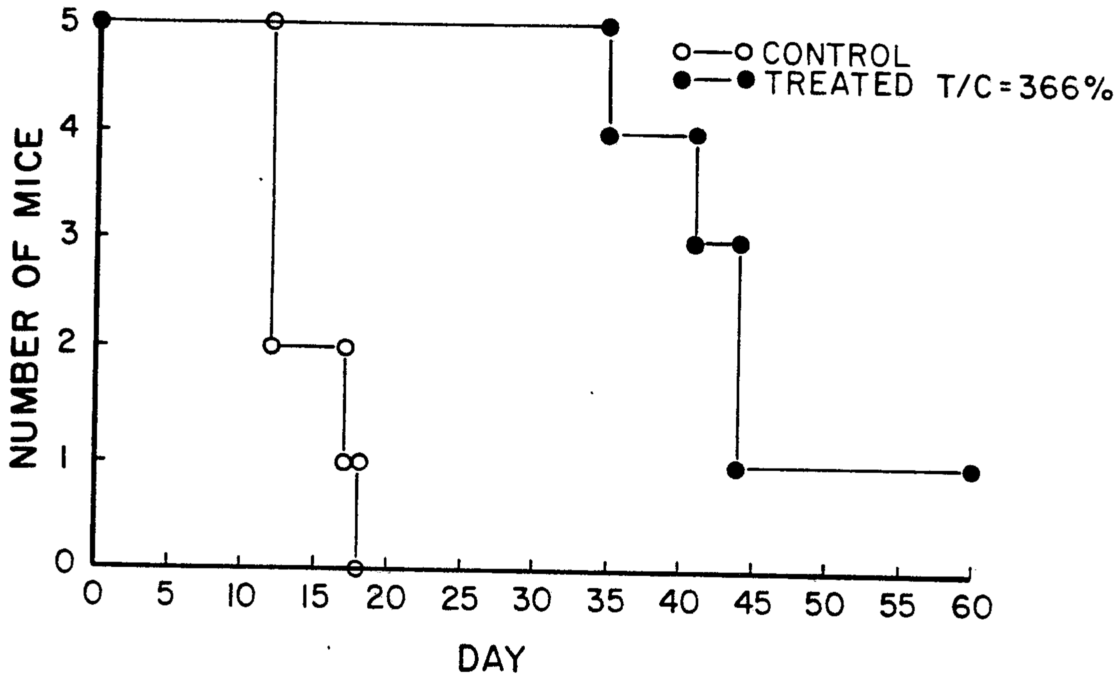

5
4
3
2
1
0
NUMBER OF MICE
0 5 10 15 20 25 30 35 40 45 50 55 60
DAY
CONTROL
TREATED T/C=366%


FIG. 1

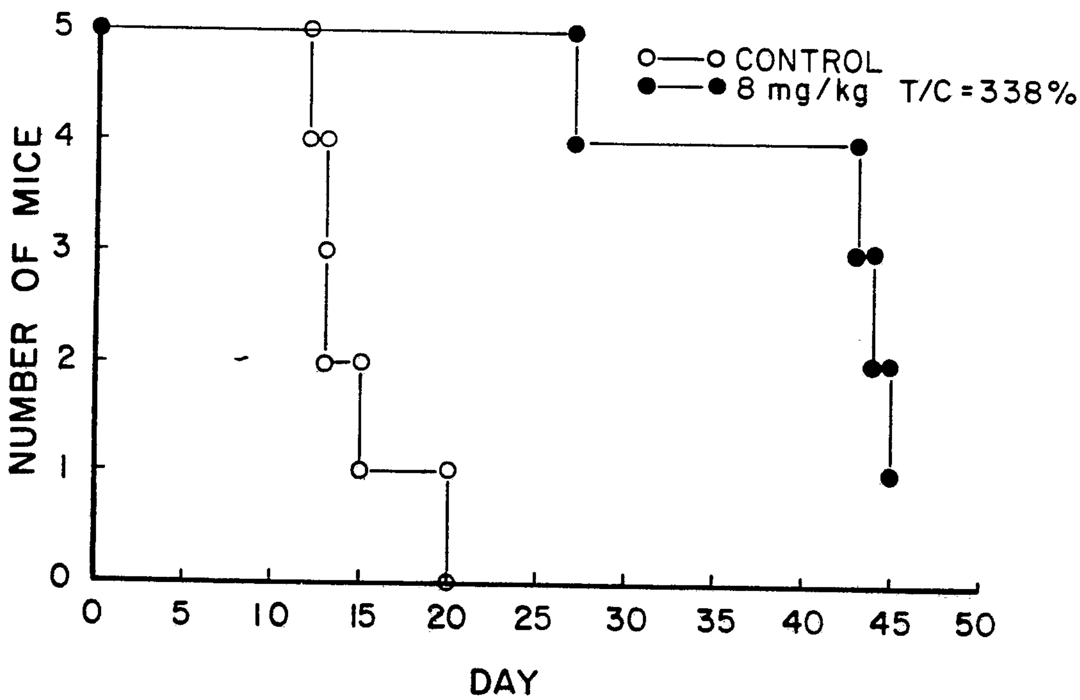

5
4
3
2
1
0
NUMBER OF MICE
0 5 10 15 20 25 30 35 40 45 50
DAY
CONTROL
8 mg/kg T/C=338%


FIG. 2

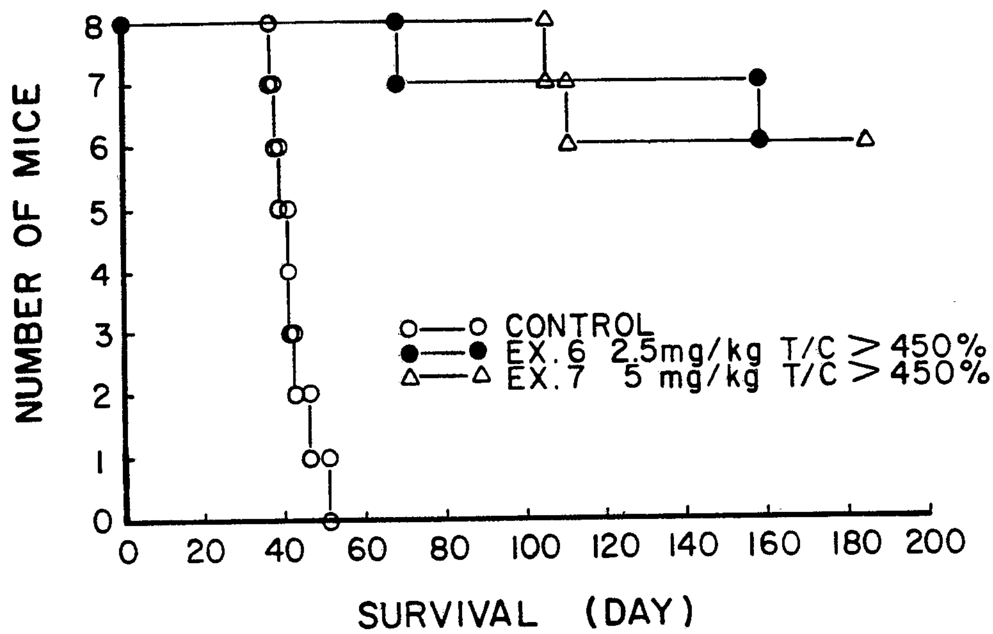
NUMBER OF MICE
8
7
6
5
4
3
2
1
0
CONTROL
EX. 6 2.5mg/kg T/C > 450%
EX. 7 5 mg/kg T/C > 450%
0 20 40 60 80 100 120 140 160 180 200
SURVIVAL (DAY)

FIG. 3

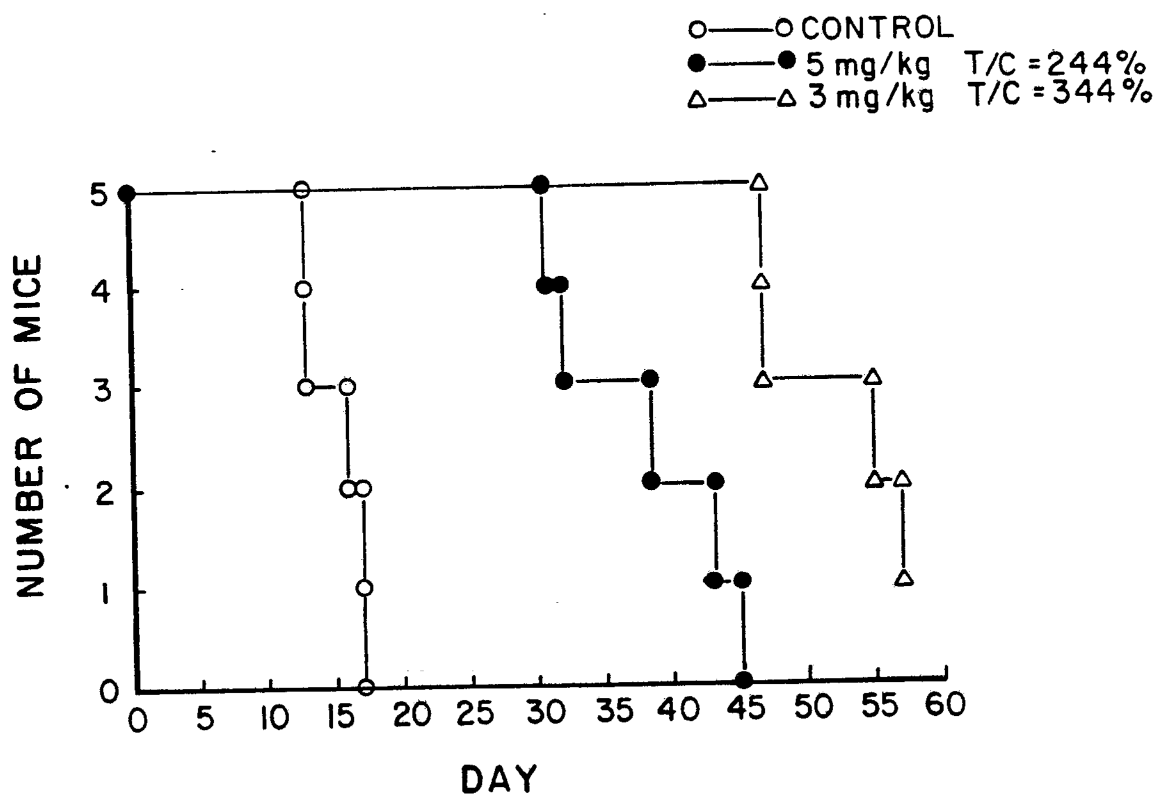
CONTROL
5 mg/kg T/C = 244%
3 mg/kg T/C = 344%
NUMBER OF MICE
5
4
3
2
1
0
0 5 10 15 20 25 30 35 40 45 50 55 60
DAY

FIG. 4